# EUROPEAN PATENT APPLICATION

(11) **EP 1 935 361 A1**
(43) Date of publication of application: **25.06.2008**
(21) Application number: 07254938.9
(22) Date of filing: 19.12.2007
(51) Int. Cl.: A61B 17/72

(54) **Bone fixing system**

(30) Priority: 19.12.2006 US 612756
(71) Applicant: Zimmer Technology, Inc., Warsaw, IN 46580 (US)
(72) Inventor: Longsworth, Serene H., Warsaw, Indiana 46580 (US); Wack, Michael A., Warsaw, Indiana 46580 (US)
(74) Representative: Mays, Julie

(57) **Abstract**

A system for fixing bone using an intramedullary nail (12).

## Description

### BACKGROUND AND SUMMARY

The present invention relates to systems for fixing bone; and, more particularly, to a system for fixing bone using an intramedullary nail.

It is known to use intramedullary nails for bone fracture repair. Screws are used to fix the position of the intramedullary nail relative to the bone.

One embodiment of the present invention provides a bone fixing system including a nail, a first screw, a second screw, and a third screw. The nail has a proximal end, a distal end, and first and second bores positioned in the distal end. The nail defines a longitudinal axis and the first and second bores are parallel. The first screw is sized and shaped to be received in the first bore of the nail. The second screw is sized and shaped to be received in the second bore of the nail. The third screw is received by the distal end of the nail and oriented obliquely relative to the longitudinal axis of the nail.

Another embodiment of the present invention provides a femur fixing system including a femoral nail, a first fastener, a second fastener, and a third fastener. The femoral nail defines a longitudinal axis and includes first and second bores defined therein. The first fastener is positioned in the first bore and fixed to a distal end of a femur. The first fastener has a first orientation relative to the femoral nail. The second fastener is positioned in the second bore and fixed to the distal end of the femur. The second fastener has a second orientation relative to the femoral nail that is parallel to the first orientation. The third fastener is fixed to the distal end of the femur. The third fastener has a third orientation that is oblique relative to the longitudinal axis of the femoral nail.

Yet another embodiment of the present invention provides a method of fixing a bone including the steps of: placing a nail within the bone, the nail including a longitudinal axis and a plurality of bores; placing a first fastener through the bone such that the first fastener passes through one of the plurality of bores of the nail, the first fastener having a first orientation relative to the longitudinal axis when placed within the bone; placing a second fastener through the bone such that the second fastener passes through one of the plurality of bores of the nail, the second fastener having a second orientation relative to the longitudinal axis when placed within the bone, the first and second orientations being substantially similar; and placing a third fastener through the bone such that the third fastener passes through one of the plurality of bores of the nail, the third fastener having a third orientation that is oblique relative to the longitudinal axis of the nail when placed within the bone.

Another embodiment of the present invention provides a femur fixing system comprising: a femoral nail having a distal end including a plurality of bores, and four screws, each screw being received in one of the plurality of bores of the femoral nail and each screw being coupled to a distal end of a femur.

### BRIEF DESCRIPTION OF THE DRAWINGS

The above-mentioned and other features of the disclosure, and the manner of attaining them, will become more apparent and better understood by reference to the following description of an embodiment of the disclosure taken in conjunction with the accompanying drawings, wherein:

Figure 1 is a side view of an intramedullary nail affixed by a plurality of screws according to the present disclosure;

Figure 2 is a top view of the affixed nail of Figure 1; and

Figure 3 is a perspective view of the affixed nail of Figure 1.

Corresponding reference characters indicate corresponding parts throughout the several views. The exemplification set out herein illustrates one embodiment of the present disclosure, in one form, and such exemplification is not to be construed as limiting the scope of the invention in any manner.

### DETAILED DESCRIPTION

The description that follows refers to a retrograde femoral nail application. While described with respect to a retrograde femoral nail application, the principles of the present disclosure can be applied to other surgical applications.

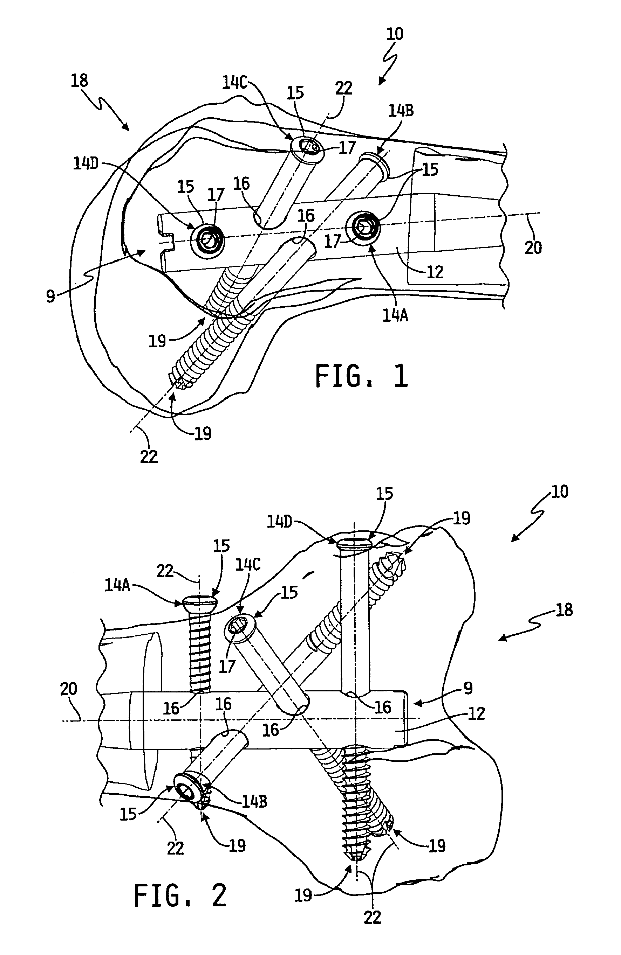

Figure 1 shows femur 10 having undergone a retrograde femoral nailing for the treatment of a femoral fracture. The procedure utilizes intemedullary nail 12 having a proximal end (not shown) and distal end 9. Nail 12 is available in a plurality of diameters such that one may be selected that is appropriate for the anatomy of the intended patient.

Placement of nail 12 in a patient having a femoral fracture includes several steps, not all of which will be discussed herein, but are known to those of ordinary skill in the art. The patient is placed in the supine position with the knee flexed approximately 30 degrees. Before or after such placement and before beginning the surgical procedure, the fracture is reduced.

A medial parapatellar incision is made in line with the femoral shaft. Next, a proper entry portal is located utilizing a Steinmann pin (not shown) and one or more radiological views. When a desired position is confirmed, a reamer (not shown) is placed over the pin and utilized to create the entry portal to the intramedullary canal. The pin and reamer are subsequently removed.

A ball-tip guide wire (not shown) and wire-grip T-handle assembly (not shown), together referred to as a guide-handle assembly, is inserted through the entry hole, into the distal canal, past the fracture site, into the proximal canal, and to a final position. The length of penetration by the guide-handle assembly is assessed to determine a proper nail length. Other methods that accurately determine the proper nail length can alternatively be used.

The wire-grip T-handle assembly is removed and an intramedullary reamer (not shown) is placed over the guide wire. Reaming is then performed along the canal in a way customized appropriately to the anatomy of the patient. The reamer is then removed.

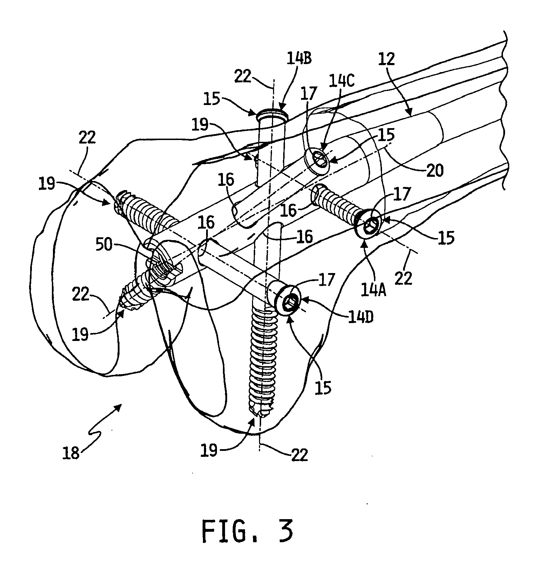

The selected nail 12 is attached to a handle/targeting device (not shown) and then placed over the guide wire and into femur 10. Once inserted, the targeting guide is used to assist in drilling transverse bores in distal end 18 of femur 10 that align with transverse bores 16 within nail 12. The targeting guide is also used to insert a plurality of locking (condyle) screws 14a-d through transverse bores 16 to secure nail 12 to distal end 18 of femur 10, as shown in Figs. 1-3.

Condyle screws 14a-d include head ends 15 having driving surfaces 17 defined therein and opposite ends 19. In one embodiment, condyle screws 14a-d are installed such that proximal-most screw 14a is installed first, with subsequent screws 14b-d being installed in order from proximal to distal. Other embodiments provide for screws 14a-d to be installed in any desired order.

Once installed, screw 14a follows a lateral-medial path. Once screw 14a is installed, screw 14d is optionally locked in place. Locking is achieved through any number of known techniques. Such locking techniques include using screws or clamps located within and axially adjustable relative to longitudinal bore 50 of nail 12, as described in U.S. Patent Application No. 2005/0101958 (the disclosure of which is expressly incorporated herein), expandable screws, expandable collets, fully-threaded nails, interference devices, or any other suitable devices for achieving the relative fixation of nail 12 and screws 14a-d.

Screws 14b, 14c are obliquely inserted into bores 16 and optionally locked in place. Screw 14d is inserted lateral-medially and optionally locked in place. Depending on the thickness of femur 10 and the length of screws 14a, 14d, screws 14a, 14d may extend out a far side of femur 10 and may be received within a nut (not shown). Accordingly, nail 12 is fixed relative to femur 10 and prevented from moving along longitudinal axis 20 of nail 12 or along longitudinal axis 22 of any screws 14a-d.

While this invention has been described as having exemplary designs, the present invention may be further modified within the spirit and scope of the disclosure. This application is therefore intended to cover any variations, uses, or adaptations of the invention using its general principles. Further, this application is intended to cover such departures from the present disclosure as come within known or customary practise in the art to which this invention pertains.

Preferred methods for using the bone fixing system of the invention to fix a bone comprise the steps of:
Placing a nail within the bone, the nail including a longitudinal axis and plurality of bores;
Placing a first fastener through the bone such that the first fastener passes through one of the plurality of bores of the nail, the first fastener having a first orientation relative to the longitudinal axis when placed within the bone;
Placing a second fastener through the bone such that the second fastener passes through one of the plurality of bores of the nail, the second fastener having a second orientation relative to the longitudinal axis when placed within the bone, the first and second orientations being substantially similar; and
Placing a third fastener through the bone such that the third fastener passes through one of the plurality of bores of the nail, the third fastener having a third orientation that is oblique relative to the longitudinal axis of the nail when placed within the bone.

Preferably the step of placing a nail includes placing the nail within a femur.

Preferably the step of placing a second fastener includes placing the second fastener proximally relative to the first fastener and the step of placing a first fastener is performed before the step of placing the second fastener.

Preferably the step of placing a first fastener includes placing the first fastener in the first orientation that is substantially perpendicular to the nail.

More preferably the step of placing a second fastener includes placing the second fastener in the second orientation that is substantially perpendicular to the nail.

More preferably the second fastener defines a longitudinal axis and the step of placing a second fastener includes placing the second fastener so that the longitudinal axis of the second fastener is in a lateral-medial orientation.

More preferably the first fastener defines a longitudinal axis and the step of placing a first fastener includes placing the first fastener so that the longitudinal axis of the first fastener is in a lateral-medial orientation.

More preferably the first and second fasteners are condyle screws.

## Claims

1. A bone fixing system comprising:
a nail having a proximal end, a distal end, and first and second bores positioned in the distal end, the nail defining a longitudinal axis, the first and second bores being parallel,
a first screw sized and shaped to be received in the first bore of the nail,
a second screw sized and shaped to be received in the second bore of the nail, and
a third screw received by the distal end of the nail and oriented obliquely relative to the longitudinal axis of the nail.

2. The bone fixing system of claim 1, wherein the first and second screws have a lateral-medial orientation when received in the nail.

3. The bone fixing system of claim 1, further comprising a fourth screw sized and shaped to be received by the distal end of the nail, the fourth screw being oblique to the longitudinal axis of the nail.

4. The bone fixing system of claim 1, wherein the first and second screws are oriented substantially perpendicular to the longitudinal axis of the nail.

5. A femur fixing system comprising:
a femoral nail defining a longitudinal axis and including first and second bores defined therein,
a first fastener positioned in the first bore and fixed to a distal end of a femur, the first fastener having a first orientation relative to the femoral nail.
a second fastener positioned in the second bore and fixed to the distal end of the femur, the second fastener having a second orientation relative to the femoral nail that is parallel to the first orientation, and
a third fastener fixed to the distal end of the femur, the third fastener having a third orientation that is oblique relative to the longitudinal axis of the femoral nail.

6. The femur fixing system of claim 5, wherein the second fastener is located distally relative to the first fastener and the first fastener includes a head end including at least one driving surface and an opposite end, the opposite end extending out of the femur when fixed thereto.

7. The femur fixing system of claim 5, further comprising a fourth screw fixed to the distal end of the femur and oriented obliquely relative to the longitudinal axis of the femoral nail.

8. The femur fixing system of claim 5, wherein the first and second fasteners are oriented perpendicularly to the longitudinal axis of the femoral nail.

9. The femur fixing system of claim 5, wherein the first and second fasteners each include a longitudinal axis arranged in a lateral-medial orientation.

10. A femur fixing system comprising:
a femoral nail having a distal end including a plurality of bores, and
four screws, each screw being received in one of the plurality of bores of the femoral nail and each screw being coupled to a distal end of a femur.

11. The femur fixing system of claim 10, wherein the distal end of the femoral nail includes four bores and each of the four bores is sized, shaped, and located to receive a single one of the four screws.

12. The femur fixing system of claim 10, wherein the four screws include:
a first screw oriented perpendicularly to a longitudinal axis of the femoral nail,
a second screw oriented obliquely to the longitudinal axis of the femoral nail,
a third screw oriented obliquely to the longitudinal axis of the femoral nail, and
a fourth screw oriented perpendicularly to the longitudinal axis of the femoral nail.
